Europäisches Patentamt

European Patent Office

Office européen des brevets

(11) Publication number: **0 300 827**
**A1**

# EUROPEAN PATENT APPLICATION

(21) Application number: **88306790.2**

(22) Date of filing: **22.07.88**

(51) Int. Cl.⁴: **A 01 G 7/00**

(30) Priority: **22.07.87 IL 83289**

(43) Date of publication of application:
**25.01.89 Bulletin 89/04**

(84) Designated Contracting States:
**AT BE CH DE ES FR GB GR IT LI LU NL SE**

(71) Applicant: **P.B. Ind. Plant Biotech Industries Ltd.**
**Mobile Post**
**Ashrat 25201 (IL)**

(72) Inventor: **Levin, Robert**
**7 Grinbaum Street**
**Nahariya (IL)**

**Hirsch, Stanley**
**Kibbutz Tuval**
**D.N. Maale Hagalil (IL)**

**Denoor, Yoram**
**Moshav Yodfat**
**D.N. Misgav (IL)**

**Poles, Nethanel**
**33 Habanim Street**
**Ramat Hasharon (IL)**

**Weiss, Nisan**
**38/19 Ben Zvi Street**
**Kiriat Haim (IL)**

**De-Nola, David**
**Kibbutz Beit Ha'emek**
**D.N. Maale Hagalil (IL)**

(74) Representative: **Lamb, John Baxter et al**
**MARKS & CLERK 57/60 Lincoln's Inn Fields**
**London WC2A 3LS (GB)**

(54) **Apparatus and method for plant growth and development.**

(57) Apparatus for the uniform distribution of plant propagules in a culture vessel, comprises blender apparatus (19) for receiving plant material and including apertured walls (23) having openings of a first size gauge for permitting plant material of less than the first size to pass therethrough, and a sieving chamber (9) enclosing the blender apparatus and including a sieving screen (15) having openings of a second size gauge smaller than the first size gauge, for permitting plant debris and liquid to drain from propagable plant material within a desired size range limited by the first and second size gauges.

EP 0 300 827 A1

**Description**

## APPARATUS AND METHOD FOR PLANT GROWTH AND DEVELOPMENT

The present invention relates to plant growth and development apparatus generally.

There is described in applicant's published European Patent Applications 0132414, 0132413, and 0232628, published on March 13, 1985, January 30, 1985 and August 19, 1987, respectively, a method of growing micro-propagated plant material whereby the plant material is supported on a screen over a culture medium.

The conventional procedure for plant tissue culture propagation is widely practiced and extensively reviewed in the literature. For example, current practices are described by P.F. Wetherall, "In Vitro Propagation" (Avery, 1982), B.V. Conger, Ed.; T.A. Thorpe, "Plant Tissue Culture - Methods and Applications in Agriculture" (Academic Press, 1981). Simply viewed, in vitro propagation consists of aseptically removing a portion of a plant and embedding it in a nutrient medium which causes growth and/or proliferation to occur. More particularly, plant tissue culture can be divided into a sequence of three major stages. The objective of the first stage is the initiation of rapidly developing aseptic culture. This culture can be initiated from a number of plant organs.

Typically, plants are surface disinfected and then handled under sterile conditions. After disinfection, explants are rinsed several times with sterile distilled water and cut into desired segments with sterile instruments. The explant is then placed on medium in a culture vessel in a culture room in which light quantity, photoperiod, and temperature are controlled.

After approximately six to eight weeks in the culture room the developed explant material is used for the initiation of the multiplication stage of in vitro propagation. Here the developed explant is aseptically removed from its vessel and trimmed and divided if necessary. The tissue is then placed in a culture vessel in a medium with the appropriate hormone composition and balance to induce multiplication and returned to the culture room. After spending another approximately six to eight weeks in the culture room on a multiplication medium, the tissue is aseptically removed from its vessel, divided, transferred to fresh medium, and returned to the culture room. This fresh medium may be a growth medium, which is formulated to induce development of shoots, with or without roots, that will withstand the transition to a greenhouse environment, or another multiplication medium, for further propagation.

The ability to recycle tissue culture repeatedly through the multiplication stage (Stage II) accounts for the rapid increase which is one of the main advantages of in vitro propagation. This procedure, however, is a very labor intensive activity. It has been estimated that as much as 60-65 per cent of the cost of producing plants in vitro can be attributed to wages, salaries and associated costs (W. C. Anderson, G.W. Meagher and A.G. Nelson, "Cost of Propagation of Broccoli Plants through Tissue Culture", Hortscience 12: 543-544, 1972); (A. Donnnan, Jr., S. E. Davidson and C. L. Williams, "Establishment of Tissue Grown Plants in the Greenhouse Environment", Proc. Fla. State. Hort. Soc. 91: 235-237, 1978). Consequently, there have been various attempts to reduce labor related expenses.

One such successful attempt is described in the aforementioned Published European Patent Application No. 0132414, the teachings of which are incorporated herein by reference.

## SUMMARY OF THE INVENTION

The present invention seeks to provide a mechanized system and technique for uniform distribution of plant propagules in a culture vessel.

There is thus provided in accordance with a preferred embodiment of the present invention apparatus for uniform distribution of plant propagules in a culture vessel, comprising blender apparatus for receiving plant material and including apertured walls having openings of a first size gauge for permitting plant material of less than a first size to pass therethrough, and a sieving chamber enclosing the blender apparatus and including a sieving screen having openings of a second size gauge smaller than the first size gauge, for permitting plant debris and liquid to drain from propagable plant material within a desired size range limited by the first and second size gauges.

There is additionally provided in accordance with a preferred embodiment of the present invention apparatus for receiving propagable plant material from the sieving chamber and concentrating a known quantity of the propagable plant material and apparatus for providing a desired dilution of the propagable plant material to provide a dilute mixture of propagable plant material.

Additionally in accordance with a preferred embodiment of the present invention there is also provided apparatus for causing a uniformly distributed dilute mixture of propagable plant material to impinge on a plant propagation matrix element, whereby the liquid passes therethrough.

Further in accordance with an embodiment of the invention, there is also provided apparatus for supplying liquid to the blender apparatus during operation thereof.

Additionally in accordance with an embodiment of the invention, there is provided apparatus for vibrating the sieving chamber.

Further in accordance with an embodiment of the present invention, there is provided apparatus for stirring the dilute mixture of propagable plant material.

## BRIEF DESCRIPTION OF THE DRAWINGS

The present invention will be understood and appreciated more fully from the following detailed description taken in conjunction with the drawings in which:

Fig. 1 is a side view illustration of apparatus for uniform distribution of plant propagules in a culture vessel constructed and operative in accordance with a preferred embodiment of the present invention;

Figs. 2A, 2B and 2C are illustrations of a plug valve useful in a modified version of the apparatus of Fig. 1, in respective first, second and third operative orientations;

Fig. 3 is a front view illustration of apparatus for dispensing plant material onto a growth matrix constructed and operative in accordance with a preferred embodiment of the present invention;

Fig. 4 is a partial cross-sectional view to a larger scale of the lower vessel portion of the apparatus of Fig. 3;

Fig. 5 is a partial cross-sectional view to a larger scale of the upper vessel portion of the apparatus of Fig. 3;

Fig. 6 is a top view of the apparatus of Fig. 3 in cross section along plane VI - VI of Fig. 3;

Fig. 7 is a top view of a first stage growing matrix and its frame;

Fig. 8 is a side view of part of the apparatus of Fig. 3, in partial cross-section along plane VIII - VIII, showing the vessel in the closed state;

Fig. 9 is a top view of the cell structure of the first stage growing matrix according to a preferred embodiment of the present invention;

Fig. 10 is a view in partial cross-section along plane X - X of the cell structure as shown in Fig. 9;

Fig. 11 is an elevational view in partial cross-section of the tissue culture vessel according to a preferred embodiment of the present invention having the first stage growing matrix inserted therein; and

Fig. 12 is a top view of the tissue culture vessel of Fig. 11 with the lid removed and the first stage growing matrix in position.

## DETAILED DESCRIPTION OF A PREFERRED EMBODIMENT

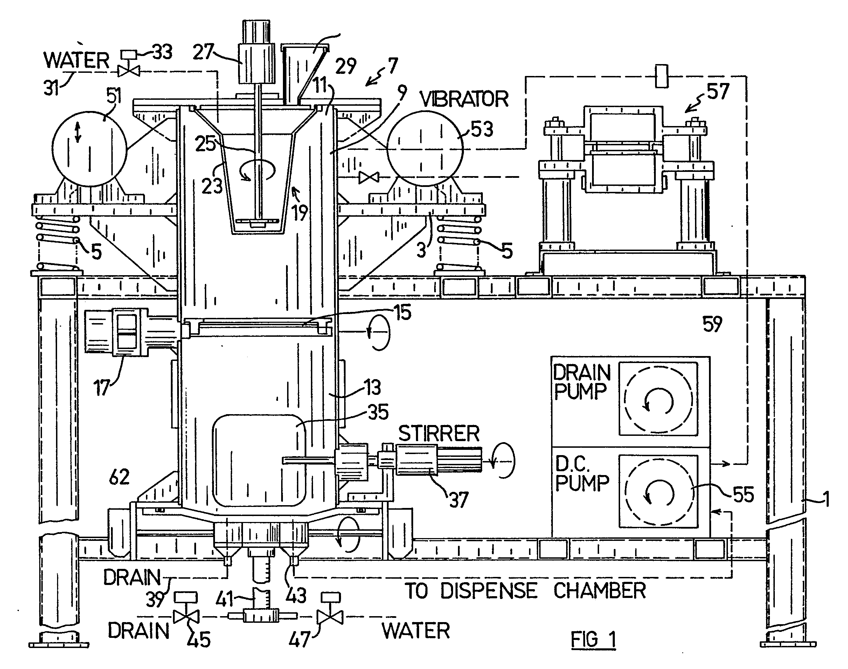

Reference is now made to Fig. 1, which illustrates compact plant propagation apparatus constructed and operative in accordance with a preferred embodiment of the present invention. The apparatus of Fig. 1 comprises a base 1 onto which is mounted a vibratable chassis 3 by means of springs 5.

Mounted on vibratable chassis 3 is a chopping and sieving assembly 7 which includes a sieving chamber 9, which is divided into top and bottom portions 11 and 13 by means of a rotatable screen 15, operated by a power drive 17.

Disposed in the top portion 11 of sieving chamber 9 is a blender assembly 19 including a container 23, at least part of the walls of which are formed of a screen or similar material having apertures of a first size gauge. The blender assembly 23 also comprises a rotatable knife assembly 25 which is typically driven by an electric motor 27. An inlet 29 for receiving plant material to be chopped is provided in communication with container 23 as is a water inlet 31, governed by a valve 33.

Disposed in the bottom portion 13 of sieving chamber 9 is a stirrer 35 which is typically powered by an electric motor 37. Disposed in fluid communication with the interior of the bottom portion 13 of sieving chamber 9 is a drain 39, a graduated measurement chamber 41 and a plant material outlet 43. Communication between the bottom portion 13 and the above-mentioned three elements is governed by a conventional selector valve (not shown).

The measurement chamber 41 communicates via valves 45 and 47 respectively with a drain and a water supply.

Apparatus for producing vibration, typically in the form of a pair of eccentric drives 51 and 53 is provided on the vibratable chassis 3.

Plant material outlet 43 communicates via a pump 55 with dispensing apparatus 57, which is described in detail hereinbelow.

The operation of the apparatus described hereinabove will now be described briefly. Generally speaking, the apparatus of Fig. 1 is operative to receive plant material, chop it into desired dimensions and dispense it onto matrices.

Plant material may be manually or automatically fed to the blending apparatus 19 via inlet 29. Water from water source 31 is supplied via valve 33 and sieving chamber 9 is thus filled with water. Plant material which has been chopped to below the first size gauge defined by the apertures of the wall of container 23 is forced out through such apertures by the action of rotating blade assembly 25. Plant material of a desired size range defined to lie between the first size gauge and the second size gauge of the screen 15 collects on screen 15. Material of smaller size, such as plant debris, whose presence is detrimental to plant development, passes through screen 15 and flows to drain 39, when liquid is removed from the chamber by means of a drain pump 59.

The plant material of a desired size range, hereinafter termed, propagable plant material, which collects on screen 15, is flushed into the bottom portion 13 of sieving chamber 9 by a flow of water from source 31, following rotation of screen 15 by about 180 degrees, as by a power drive 17.

The propagable plant material is concentrated in a measurement chamber 41 for measurement of its quantity. It is then returned to the bottom portion 13 of sieving chamber 9 by means of a flow of water via valve 47 and is combined with a known quantity of water, to provide a mixture of plant material and water of desired dilution. This mixture is rendered uniform by means of a stirrer 35, driven by motor 37.

The uniformized mixture is fed via outlet 43 and pump 55 to dispensing apparatus 57, the operation of which will be described hereinbelow. Meanwhile, screen 15 is returned to its original horizontal orientation.

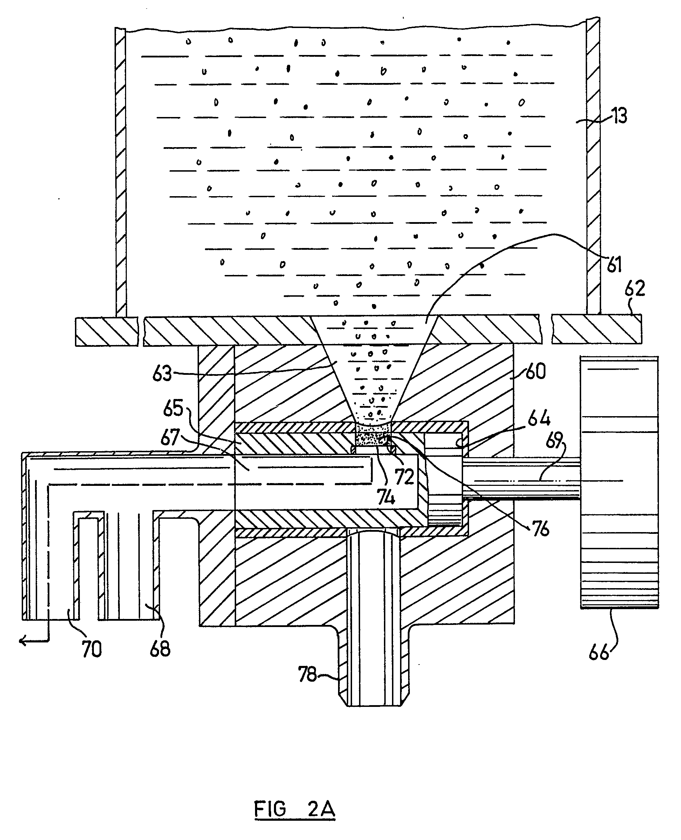

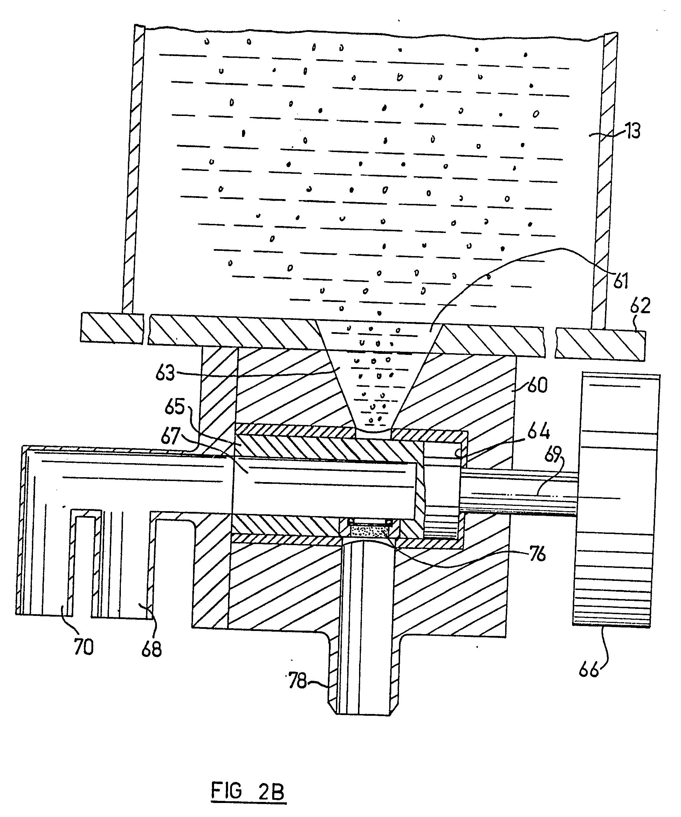

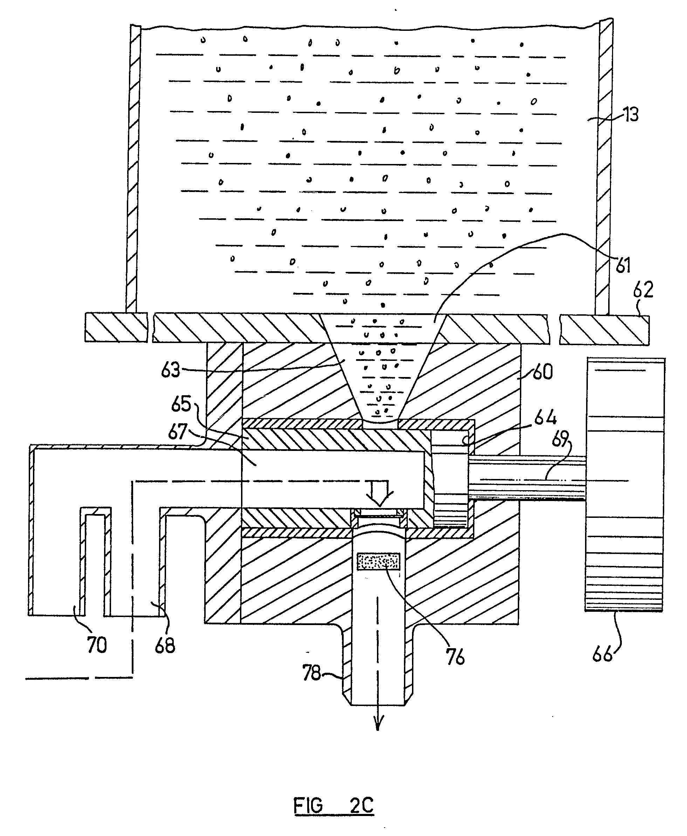

According to an alternative embodiment of the present invention, the measurement chamber 44 and associated apparatus may be replaced by a plug valve assembly, which is illustrated in Figs. 2A, 2B and 2C.

The apparatus illustrated in Figs. 2A, 2B and 2C comprises a housing 60, which is fixedly mounted onto the bottom of the bottom portion 13 of sieving chamber 9 and communicates therewith via an opening 61 formed in the bottom wall 62 thereof.

Housing 60 defines a funnel type inlet 63 which communicates with a cylindrical bore 64 in which is located a rotatable cylindrical element 65, typically formed or coated with a plastic material, such as teflon, and to which is attached a handle 66 for rotation of the plug about an axis 69.

The element 65 is formed with an internal axial passageway 67 which communicates with a water inlet 68 and a drain 70. Passageway 67 communicates with a concentrating cup 72 in which is disposed a screen 74, at a position recessed with respect to the outer cylindrical surface of the element 65.

The screen 74 and cup 72 are arranged such that when the element 65 is in its first operative orientation, illustrated in Fig. 2A, dilute plant material from bottom portion 13 of chamber 9, passes into cup 72, such that the liquid passes through the screen 74 to drain 70 and the solid plant material is compacted into a plug 76 of predetermined volume defined by the volume of the cup 72 between the screen 74 and the outer cylindrical surface of the element 65.

Rotation of element 65 by 180 degrees, as illustrated in Fig. 2B, seals cup 72 from chamber portion 13 and brings the plug 76 into communication with an outlet 78. A measured quantity of water may then be supplied under pressure via inlet 68 and passageway 67 to propel plug 76, as illustrated in Fig. 2C, to dispensing apparatus 57 in precisely measured dilute form.

The above-described apparatus may be manually operated or alternatively it may be provided with an automatic control based on an SMC ECC 50 controller.

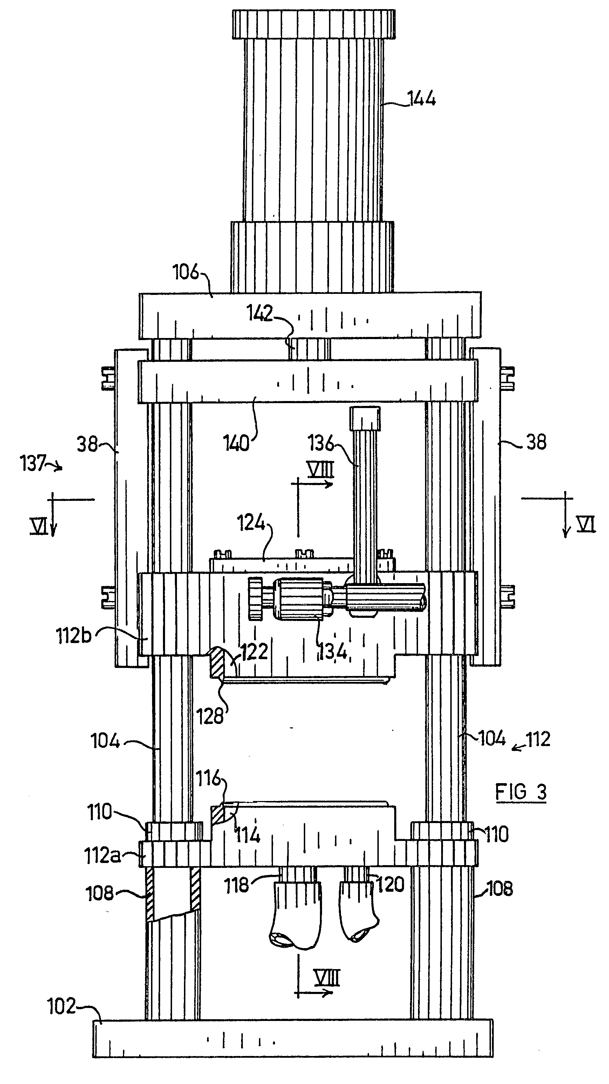

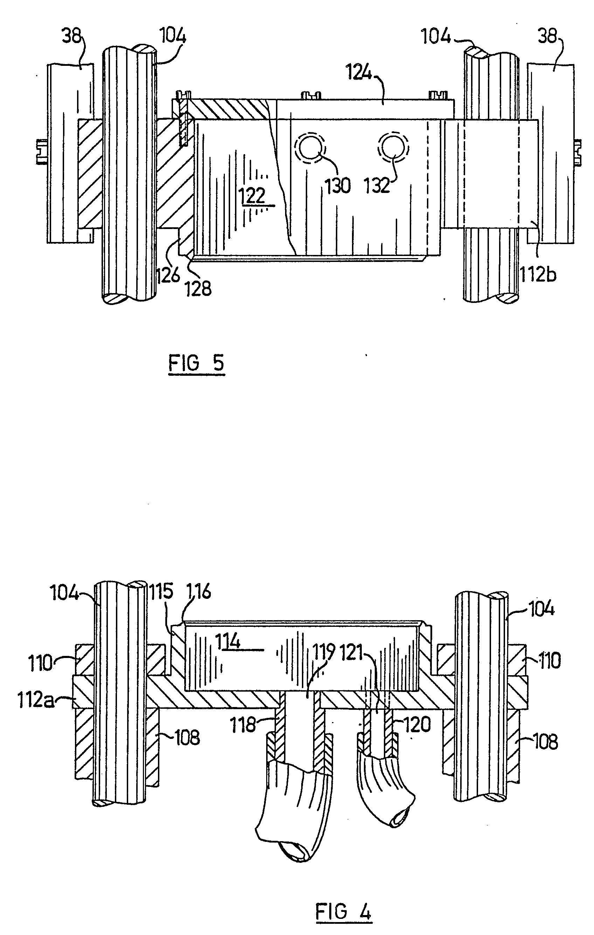

Dispensing apparatus 57 is illustrated in Fig. 3 and comprises a base 102, onto which are fixedly mounted two columns 104 connected on their upper ends by a cross member 106. Seated on two sleeves 108 slipped over the columns 104, and held in position by clamping rings 110, is the lower stationary portion 112a of a sealable two-part vessel 112 preferably made of stainless steel. This lower portion 112a is shown at a larger scale in Fig. 4 and is seen to comprise a lower chamber 114 defined by a recess of, as seen from above, a substantially square cross section.

The rim 115 of the chamber 114 is provided with a knife edge-type sealing lip 116 surrounding the chamber 114 on all sides. Attached to the bottom of chamber 114 are, in the center, a first rubber-tube connector 118 constituting a drain port 119 and, somewhat off center, a second rubber-tube connector 120 constituting an air inlet port 121, the first serving as a connector for a drain tube leading to a sink or the like, the second as a connector to a source of compressed air. Both tubes are controllable by pinch cocks or valves (not shown).

Fig. 5 shows the upper, movable portion 112b of the vessel 112, comprising an upper chamber 122 constituted by an opening of substantially square cross-section, and tightly covered by a transparent cover plate 124 made of Perspex or a similar material.

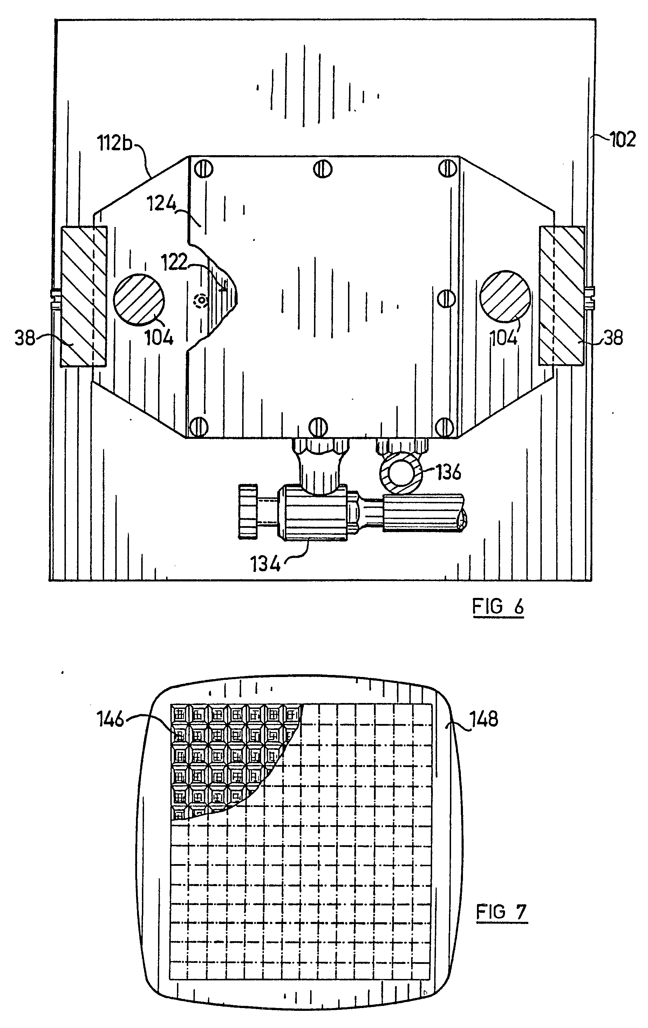

The rim 126 of the chamber 122 is provided with a knife-edge type sealing lip 128 surrounding the rim, similar to the sealing lip 116 of the lower vessel portion 112a. Two threaded holes, 130 and 132, lead into chamber 122, the first, 130, serving to connect a valve 134 controlling the inflow of the above-mentioned plant propagule/liquid mixture, and the second, 132, for connection of an air relief valve 136, the purpose of which will become apparent hereinbelow. Valves 134 and 136 are illustrated in Figs. 3 and 6.

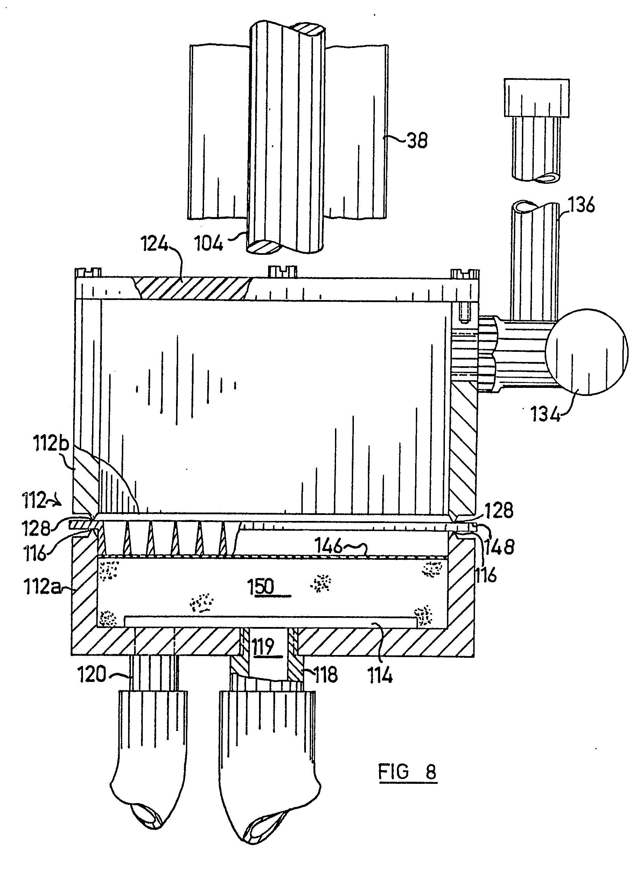

The upper vessel portion 112b is slidably mounted on the columns 104. At the same time it constitutes the lower cross member of a frame 137 (Fig. 3) comprising two uprights 38 to the lower ends of which it is fixedly attached, and an upper cross member 140 (Fig. 3) to which is attached the piston rod 142 of a pneumatic cylinder 144 mounted on the cross member 106. By actuating the cylinder 144, the frame 137 can be raised, as seen in Fig. 3, or lowered, so that the upper vessel portion 112b approaches the lower portion 112a as seen in Fig. 8, to be explained further below.

Fig. 6 shows the outline of the upper vessel portion 112b as seen in top view, the outline of the lower vessel portion 112a being identical.

A first stage growing matrix 146, and its frame flange 148 are shown in Fig. 7. Another view of this matrix is afforded in Fig. 8, which will be explained below.

Fig. 8 illustrates the two-part vessel 112, in the closed, working position in which, by actuating the pneumatic cylinder 144 as mentioned above, the upper vessel portion 112b has been moved downwards and brought to bear against the flange 148 of the matrix 146, which has been inserted into the lower chamber 114. The purpose of sealing lips 116 and 128 is now clear: being relatively sharp, they slightly dent the surfaces of the nylon flange 148, thus producing a tight seal.

Further seen in Fig. 8 is a sparger 150 typically comprising a sintered, porous metal block with a mean pore size of about 35 microns, the purpose of which is to provide an even flow of minute air bubbles through the above-mentioned plant propagule/liquid mixture, thereby ensuring a uniform distribution of the propagules in the liquid, as explained below.

Broadly, the preferred process of an embodiment of the present invention is as follows: The vessel 112, which has been sterilized and maintained in a sterile atmosphere, is closed and sealed by the pneumatic piston, the inlet valve 134 to the upper vessel portion 112b is opened, and a fixed amount of sterile plant material and liquid is pumped into the vessel 112 so that the vessel is nearly filled. The drain port 119 is closed. At the same time, the air valve 136 is opened. Liquid now fills the entire vessel 112 both above and below the matrix, however, the plant material is retained by the matrix 146 in the upper chamber.

Sterile air, under a pressure of 2-3 atmospheres, rises evenly from the sparger 150 in small bubbles, thoroughly mixing the plant material in the liquid above the screen 146. After about 20 seconds of mixing, the air valve 136 is closed and the drain valve is opened. The fluid slowly drains from the vessel 112 leaving the plant material evenly distributed on the matrix. The vessel 112 is then opened by pneumatically raising the upper portion 112b, and the matrix 146 and its frame 148 are aseptically removed and placed in a culture vessel on top of and in good contact with a medium base which is the optimal configuration for growth and development of the tissue into plantlets. The plantlets are then transplanted to transplanting trays by apparatus and using a technique which is described hereinbelow.

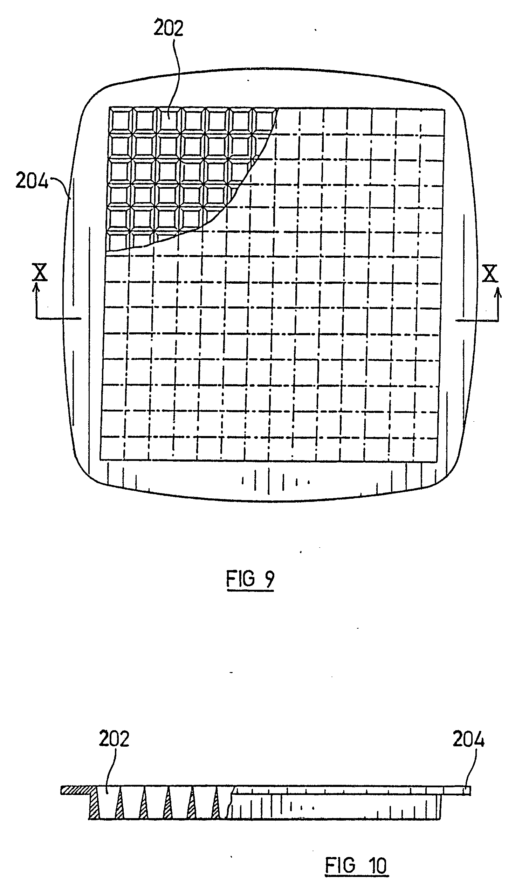

Referring again to the drawings, there is seen in Figs. 9 and 10 the matrix structure according to the invention, advantageously made of a sterilizable material such as one of the nylon groups of plastics, comprising a grid-like array of cells 202 having a substantially square cross-section and being open at the top. The cell walls are solid and converge downwardly such that the cells define inverted truncated pyramids, with each two adjacent walls of each two adjacent cells 202 meeting at the upper surface of the matrix to form a substantially sharp edge, as is clearly seen in Fig. 10. The upper surface of the matrix is provided with a plane rim 204 which fits the tissue culture vessel, discussed further below, with clearance.

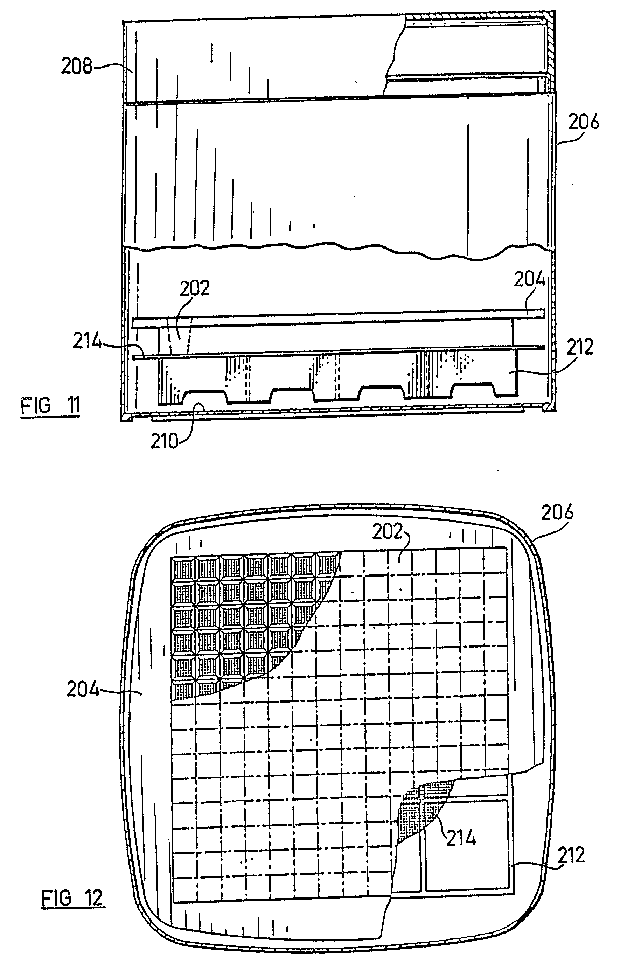

In Figs. 11 and 12, the cell structure according to an embodiment of the invention is shown in position inside the culture vessel 206, the top of which is shown as closed by means of a lid 208. On the bottom 210 of the vessel 206, is disposed a grating-like supporting member or pallet 212, about 1 cm high, on which rests a substantially flat nylon screen 214 which, as has been explained above, allows the culture medium to pass through, but has a mesh fine enough to retain the tissue culture inside the cells 202 of the cell structure mounted on the screen 214. A preferred mesh size is 500 to 700 microns, an acceptable mesh size being 250 - 4000 microns.

While in the embodiment shown the screen 214 is a separate entity, a variant can be envisaged in which the screen 214 is an integral part of the matrix, being, e.g., molded to the bottom surface thereof.

It will be appreciated by persons skilled in the art that the present invention is not limited by what has been particularly shown and described hereinabove. Rather the scope of the present invention is defined only by the claims which follow:

**Claims**

1. Apparatus for uniform distribution of plant propagules in a culture vessel comprising:
chopping means (19) for receiving plant material and including apertured walls (23) having openings of a first size gauge for permitting plant material of less than the first size to pass therethrough; and
a sieving chamber (9) enclosing the chopping means and including a sieving screen (15) having openings of a second size guage smaller than the first size guage, for permitting plant debris and liquid to drain from propagable plant material within a desired size range limited by the first and second size gauges.

2. Apparatus according to claim 1 also comprising means (41) for receiving propagable plant material from the sieving chamber and concentrating a known quantity of the propagable plant material and means (13, 47) for providing a desired dilution of the propagable plant material to provide a dilute mixture of propagable plant material.

3. Apparatus according to claim 1 or claim 2 also comprising means for causing a uniformly distributed dilute mixture of propagable plant material to impinge on a plant propagation matrix element, whereby the liquid passes therethrough.

4. Apparatus according to any one of claims 1 - 3 also comprising means (53) for vibrating the sieving chamber.

5. Apparatus according to any one of claims 1 - 4 also comprising means (35, 37) for stirring the dilute mixture of propagable plant material.

WATER  33  27  29  7

31  51  11  9  VIBRATOR  57  53

25  23  19  3  5

5  15  59

17  13  DRAIN PUMP

35  D.C. PUMP  55

62  STIRRER  37

DRAIN  TO DISPENSE CHAMBER

39  41  43

DRAIN  45  47  WATER  FIG 1

FIG 2A

0300827

FIG 2B

0300827

FIG 2C

0300827

FIG 3

0300827

FIG 5

FIG 4

0300827

FIG 6

FIG 7

0300827

FIG 8

0300827

FIG 9

FIG 10

0300827

FIG 11

FIG 12

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 605 173 (H.A. EDMONDS) <br> * figure 3 * | 1 | A 01 G 7/00 |
| D,A | EP-A-0 132 414 (MILOUDA LTD.) <br> * pages 15-21; figure 1 * | 1 | |
| D,A | EP-A-0 132 413 (MILOUDA LTD.) <br> * figure 2 * | 1 | |
| P,D <br> A | EP-A-0 232 628 (P.B. IND. PLANT BIOTEC LTD.) <br> * claims; figures * | 1 | |

TECHNICAL FIELDS
SEARCHED (Int. Cl.4)

A 01 G 7/00
A 01 G 31/00
B 02 C 18/00

The present search report has been drawn up for all claims

| Place of search | Date of completion of the search | Examiner |
|---|---|---|
| BERLIN | 19-10-1988 | WUNDERLICH J E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P0401)